(19) Europäisches Patentamt
European Patent Office
Office européen des brevets

(11) Veröffentlichungsnummer: **0 037 474**
**B1**

(12) **EUROPÄISCHE PATENTSCHRIFT**

(45) Veröffentlichungstag der Patentschrift: **06.04.83**

(51) Int. Cl.³: **C 07 C 69/63**, C 07 C 67/00

(21) Anmeldenummer: **81101866.2**

(22) Anmeldetag: **13.03.81**

(54) **Verfahren zur Herstellung von 1-Acetoxy-3-chlor-pentan-4-on.**

(30) Priorität: **05.04.80 DE 3013269**

(43) Veröffentlichungstag der Anmeldung: **14.10.81 Patentblatt 81/41**

(45) Bekanntmachung des Hinweises auf die Patenterteilung: **06.04.83 Patentblatt 83/14**

(84) Benannte Vertragsstaaten: **CH DE FR GB LI**

(56) Entgegenhaltungen: **keine**

(73) Patentinhaber: **BASF Aktiengesellschaft, Carl-Bosch-Strasse 38, D-6700 Ludwigshafen (DE)**

(72) Erfinder: **Littmann, Wolfgang, Dr., Neckarpromenade 36, D-6800 Mannheim (DE)**
Erfinder: **Paust, Joachim, Dr., Ringstrasse 3, D-6701 Neuhofen (DE)**

**EP 0 037 474 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 1-Acetoxy-3-chlor-pentan-4-on

Die Erfindung betrifft ein verbessertes Verfahren zur Herstellung von 1-Acetoxy-3-chlor-pentan-4-on der Formel

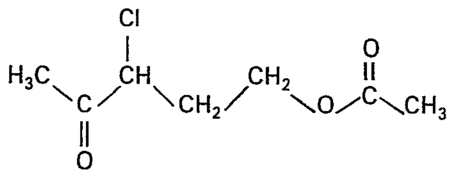

(I)

ausgehend von α-Chlor-α-aceto-γ-butyrolacton. Das Verfahrensprodukt ist bekanntlich ein wichtiges Zwischenprodukt bei der Herstellung von Vitamin $B_1$.

In der Literatur werden mehrere Methoden zur Herstellung von 1-Acetoxy-3-chlor-pentan-4-on (I) beschrieben. Das beste der beschriebenen Verfahren war das Verfahren gemäss der deutschen Patentschrift 820 297. Gemäss dieser deutschen Patentschrift lässt sich (I) dadurch herstellen, dass man α-Chlor-α-aceto-γ-butyrolacton der Formel II

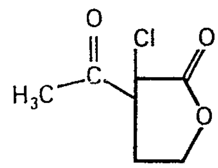

(II)

zunächst mit einem Gemisch aus Salzsäure, Essigsäure und Wasser umsetzt und das erhaltene Gemisch in einem zweiten Verfahrensschritt mit Acetanhydrid reagieren lässt.

Gemäss der genannten Patentschrift nimmt dieses Verfahren folgenden Verlauf:

1. Stufe (Hydrolyse):

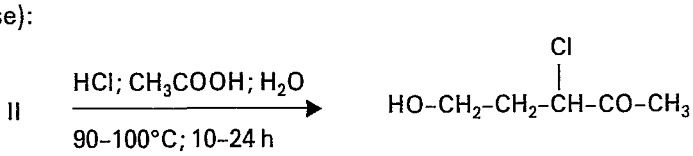


(III)

2. Stufe (Acetylierung):

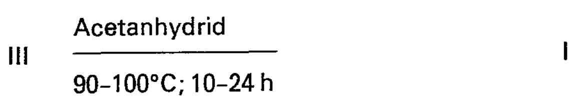


Nachteilig an diesem Verfahren sind vor allem die langen Reaktionszeiten, nämlich 10 bis 24 Stunden für die erste und 10 bis 24 Stunden für die zweite Stufe sowie ein recht erheblicher Aufwand für die destillative Abtrennung der zahlreichen Nebenprodukte bei der Aufarbeitung des Reaktionsansatzes und die Notwendigkeit der Verwendung des relativ teuren Acetanhydrids.

Dementsprechend war es Aufgabe der Erfindung I ausgehend von II unter weitgehender Vermeidung der genannten Nachteile herzustellen.

Es wurde nun gefunden, dass man 1-Acetoxy-3-chlor-pentan-4-on (I) durch Umsetzen von α-Chlor-α-aceto-γ-butyrolacton (II) mit einem Acetylierungsmittel in wässrig-salzsaurem Medium auf sehr wirtschaftliche Weise erhält, wenn man II in Gegenwart von Chlorwasserstoff bei 70 bis 100°C, vorzugsweise 85 bis 95°C 1 bis 8, vorzugsweie 1 bis 2 Stunden lang mit 0,8 bis 1,2 Mol Wasser und 1 bis 2 Mol Essigsäure pro Mol II reagieren lässt und das Reaktionsgemisch danach destillativ auf I aufarbeitet.

Dieses Verfahren ist als äusserst überraschend zu bezeichnen, da es zu den bisherigen Vorstellungen über den Reaktionsverlauf und dadurch auch zu der bisherigen Herstellungspraxis in krassem Widerspruch steht. Man ging nämlich davon aus, dass II zunächst vollständig hydrolysiert werden müsste, bevor der hierbei als Zwischenverbindung erwartete freie Alkohol III acetyliert werden kann. Diese Vorstellung führte zwangsläufig zu den langen Reaktionszeiten der beiden Verfahrensschritte sowie zu der Massnahme, die Acetylierung mittels dem starken Acetylierungsmittel Acetanhydrid vorzunehmen. Tatsächlich findet die Umsetzung von II zu dem gewünschten 1-Acetoxy-3-chlor-pentan-4-on jedoch überraschenderweise schon durch die im Reaktionsgemisch vorhandene Essigsäure statt. Acetylierungen mit Essigsäure in Gegenwart von Wasser sind jedoch absolut ungewöhnlich. Der Reaktionsmechanismus für diese Umsetzung ist noch unklar.

Wie unerwartet eine solche Verfahrensdurchführung war, wird besonders deutlich dadurch, dass das Verfahren der DE-PS 820 297 jahrzehntelang nahezu unverändert durchgeführt wurde. Dies zeigt sich beispielsweise in der US-PS 2 932 653 (patentiert 1960; vgl. Spalte 2, Zeilen 55 bis 67) und Ed. Sci. 34, 41–45 (1979).

Das als Ausgangsverbindung für das erfindungsgemässe Verfahren benötigte II ist leicht aus handelsüblichem Acetobutyrolacton herstellbar (siehe DE-OS 1 568 177) oder kann gemäss der US-PS 2 932 653 hergestellt werden.

Die Umsetzung mit Essigsäure erfolgt in wässrig-salzsaurer Lösung, die katalytische Mengen Salzsäure und etwa 1 Mol Wasser pro Mol II enthält. Unter katalytischen Mengen HCl versteht man im allgemeinen Mengen von etwa 0,1 bis 10 g, vorzugsweise 0,5 bis 1 g pro Mol II.

Die Verwendung geringer Über- oder Unterschüsse an Wasser (etwa 0,8 bis 1,2 Mol) im Reaktionsgemisch macht sich auf die I-Ausbeute nicht sehr störend bemerkbar. Verwendet man hingegen weniger als etwa 0,6 Mol Wasser pro Mol II, so werden deutlich niedrigere I-Ausbeuten erzielt. Verwendet man weniger als 0,4 Mol Wasser pro Mol II, so findet die gewünschte Reaktion kaum noch statt. Werden grössere Mengen Wasser verwendet, so bildet sich im wesentlichen der freie Alkohol III, der nur schwierig acetylierbar ist.

Das Wasser kann als solches zugesetzt werden oder aber in Form des Wassergehaltes der eingesetzten Essigsäure bzw. von Essigsäure und Salzsäure.

Die Essigsäure verwendet man in Mengen von 1 bis 2, vorzugsweise 1,3 bis 1,7 Mol pro Mol II. Bei Verwendung von mehr als 2 Mol Essigsäure pro Mol II im Reaktionsgemisch wird die gewünschte Umsetzung zusehends langsamer.

Die Reaktionstemperaturen betragen 70 bis 100°C, vorzugsweise 85 bis 95°C. Je nach Reaktionstemperatur und Wasser- bzw. Essigsäuremenge betragen die Reaktionszeiten etwa 1 bis 4 Stunden. Unter den bevorzugten Reaktionsbedingungen ist die Umsetzung bereits nach 1 bis 2 Stunden vollständig abgelaufen.

Die verhältnismässig kurzen Reaktionszeiten bringen neben dem grossen Vorteil der weitaus höheren Raumzeitausbeuten und der dadurch bedingten starken Energieeinsparung noch einen weiteren enormen Vorteil mit sich. Bei der Umsetzung werden weitaus weniger Nebenprodukte gebildet, wodurch das gewünschte Verfahrensprodukt durch einfache Destillation unter vermindertem Druck in reiner Form erhalten werden kann, während die destillative Aufarbeitung des Reaktionsgemisches, das gemäss dem Verfahren der DE-PS 820 297 erhalten wird, in der Praxis sehr aufwendig und schwierig ist.

Mit Hilfe des erfindungsgemässen verbesserten Verfahrens kann das als wichtiges Zwischenprodukt für die Herstellung von Vitamin $B_1$ benötigte 1-Acetoxy-3-chlor-pentan-4-on auf einfache und wirtschaftliche Weise hergestellt werden.

Beispiel 1

162,5 g (1 Mol) α-Chlor-α-aceto-γ-butyrolacton werden mit 78 g einer 76%igen wässrigen Essigsäure (1 Mol Essigsäure + 1 Mol $H_2O$) und 2 g konz. Salzsäure versetzt und das Gemisch 90 Minuten auf 90°C erhitzt. Das Reaktionsgemisch wird anschliessend auf Raumtemperatur abgekühlt, das Wasser-Essigsäure-Gemisch unter vermindertem Druck abgezogen und der Rückstand unter vermindertem Druck destilliert. Man erhält 151 g 1-Acetoxy-3-chlor-pentan-4-on. Das entspricht einer Ausbeute von 85% der Theorie.

Beispiel 2

162,5 g (1 Mol) α-Chlor-α-aceto-γ-butyrolacton werden mit 138 g einer 87%igen wässrigen Essigsäure (2 Mol Essigsäure + 1 Mol $H_2O$) und 2 g konz. Salzsäure versetzt und das Gemisch für 2 Stunden auf 90°C erhitzt. Das Reaktionsgemisch wird anschliessend auf Raumtemperatur abgekühlt, das Wasser-Essigsäure-HCl-Gemisch unter vermindertem Druck abgezogen und der Rückstand bei 20mbar destilliert. Man erhält 162,5 g 1-Acetoxy-3-chlor-pentan-4-on. Das entspricht einer Ausbeute von 91% der Theorie.

**Patentansprüche**

1. Verfahren zur Herstellung von 1-Acetoxy-3-chlor-pentan-4-on (I) durch Umsetzen von α-Chlor-α-aceto-γ-butyrolacton (II) mit einem Acetylierungsmittel in wässrig-salzsaurem Medium, dadurch gekennzeichnet, dass man (II) in Gegenwart von Chlorwasserstoff bei 70 bis 100°C 1 bis 8 Stunden lang mit 0,8 bis 1,2 Mol Wasser und 1 bis 2 Mol Essigsäure pro Mol II reagieren lässt und das Reaktionsgemisch danach destillativ auf I aufarbeitet.

2. Verfahren gemäss Anspruch 1, dadurch gekennzeichnet, dass man das α-Chlor-α-aceto-butyrolacton 1 bis 2 Stunden mit Wasser und Essigsäure reagieren lässt.

3. Verfahren gemäss Anspruch 1, dadurch gekennzeichnet, dass man das α-Chlor-α-aceto-butyrolacton bei 85 bis 95°C mit Wasser und Essigsäure reagieren lässt.

**Claims**

1. A process for the preparation of 1-acetoxy-3-chloro-pentan-4-on (I) by reacting α-chloro-α-aceto-γ-butyrolactone (II) with an acetylating agent in an aqueous hydrochloric acid medium, wherein (II) is reacted with from 0.8 to 1.2 moles of water and from 1 to 2 moles of acetic acid per mole of (II) in the presence of hydrogen chloride at from 70 to 100°C for from 1 to 8 hours, and the reaction mixture is then worked up by distillation to give (I).

2. A process as claimed in claim 1, wherein α-chloro-α-aceto-γ-butyrolactone is reacted with water and acetic acid for from 1 to 2 hours.

3. A process as claimed in claim 1, wherein α-chloro-α-aceto-γ-butyrolactone is reacted with water and acetic acid at from 85 to 95°C.

**Revendications**

1. Procédé pour la préparation de 1-acétoxy-3-chloro-pentanone-4 (I) par réaction d'α-chloro-α-acéto-γ-butyrolactone (II) avec un agent d'acétylation dans un milieu chlorhydrique aqueux, caractérisé en ce que l'on fait réagir (II) avec 0,8 à 1,2 mol d'eau et 1 à 2 mol d'acide acétique par mol de II, en présence d'acide chlorhydrique, à une température de 70 à 100°C, pendant 1 à 8 heures.

2. Procédé selon la revendication 1, caractérisé en ce qu'on fait réagir l'α-chloro-α-acéto-γ-butyrolactone pendant 1 à 2 heures avec l'eau et l'acide acétique.

3. Procédé selon la revendication 1, caractérisé en ce qu'on fait réagir l'α-chloro-α-acéto-γ-butyrolactone avec l'eau et l'acide acétique à une température de 85 à 95°C.